# EUROPÄISCHE PATENTSCHRIFT

(11) **EP 0 518 321 B1**
(45) Veröffentlichungstag und Bekanntmachung des Hinweises auf die Patenterteilung: **01.02.1995**
(21) Anmeldenummer: 92109832.3
(22) Anmeldetag: 11.06.1992
(51) Int. Cl.: C07C 317/28, C07C 315/02

(54) **Verfahren zur Herstellung von 3'-Aminopropyl-2-chlorethylsulfon-semisulfat**
Process for the preparation of 3-aminopropyl-2-chloroethyl sulfone semisulfate
Procédé pour la préparation de 3-aminopropyl-2-chloroéthyl sulfone semisulfate

(30) Priorität: 12.06.1991 DE 4119287
(43) Veröffentlichungstag der Anmeldung: 16.12.1992
(73) Patentinhaber: HOECHST AKTIENGESELLSCHAFT, 65926 Frankfurt am Main (DE)
(72) Erfinder: Angenendt, Heinrich, Dr., W-6000 Frankfurt am Main 71 (DE); Meier, Michael, Dr., W-6000 Frankfurt am Main (DE)

(56) Entgegenhaltungen:
- WO-A-91/13866
- US-A- 2 824 887

## Beschreibung

Die Erfindung betrifft ein Verfahren zur Herstellung Von 3'-Aminopropyl-2-chlorethylsulfon in Form des Semisulfats in sehr guter Ausbeute und hoher Reinheit, indem man in einem Eintopfverfahren Allylamin mit Mercaptoethanol in verdünnter Schwefelsäure in Gegenwart eines Radikalstarters umsetzt, in die anfallende Mischung Chlor einleitet und die erhaltene Mischung anschließend zur Trockne einengt.

3'-Aminopropyl-2-chlorethylsulfon ist ein wichtiges Vorprodukt zur Herstellung von Reaktivfarbstoffen (EP 141 776, EP 174 909 und EP 208 655). Die Herstellung von 3'-Aminopropyl-2-chlorethylsulfon wurde in der Literatur noch nicht beschrieben. Literaturbekannt ist jedoch die Herstellung von 2'-Aminoethyl-2-chlorethylsulfon-hydrochlorid. Hierbei wird nach der US-PS 2 824 887 2-Chlorethylamin-hydrochlorid mit dem Natriumsalz von Mercaptoethanol umgesetzt und das so erhaltene 2'-Aminoethyl-2-hydroxyethylsulfid mit Thionylchlorid zum 2'-Aminoethyl-2-chlorethylsulfid-hydrochlorid chloriert und anschließend mit Peressigsäure zum 2'-Aminoethyl-2-chlorethylsulfon-hydrochlorid oxidiert. 2'-Aminoethyl-2-hydroxyethylsulfid kann auch nach literaturbekannten Verfahren (z.B. Izv. Akad. Nauk. SSSr, Ser. Khim, 1976, 4, 937-940 und EP-PS 015 929 (Seite 80/81, Beispiel 88)) durch Chlorierung und Oxidation mit Chlor zum 2'-Aminoethyl-2-chlorethylsulfon-hydrochlorid umgesetzt werden.

Wollte man 3'-Aminopropyl-2-chlorethylsulfon in analoger Weise herstellen, so wäre der Anfall von zwei Äquivalenten Natriumchlorid bei der Herstellung des 3'-Aminopropyl-2-hydroxyethylsulfids in Kauf zu nehmen, die auf der Sulfid- oder Endstufe aufwendig und verlustreich abgetrennt werden müßten.
Bei einer Chlorierung mit Thionylchlorid und anschließender Oxidation mit Peressigsäure analog der US-PS 2 824 887 würden zusätzlich noch je ein Äquivalent Schwefeldioxid und Chlorwasserstoff zwangsweise anfallen.

Somit ist festzuhalten, daß diese Verfahren den heutigen Ansprüchen an ein technisches Verfahren nicht gerecht werden.
Es bestand daher ein Bedürfnis für ein wirtschaftliches und technisch leicht durchführbares Verfahren zur Herstellung von 3'-Aminopropyl-2-chlorethylsulfon.

Es wurde nun überraschenderweise gefunden, daß man 3'-Aminopropyl-2-chlorethylsulfon in Form des Semisulfats in sehr guter Ausbeute (>95 % d.Th.) und in hoher Reinheit vorteilhaft herstellen kann, indem man in einem Eintopfverfahren Allylamin mit Mercaptoethanol in 0,5 bis etwa 0,6 mol, vorzugsweise 0,50 bis etwa 0,55 mol verdünnte (wäßrige) Schwefelsäure, bezogen auf das Allylamin, bei Temperaturen von etwa 0°C bis zum Siedepunkt der Reaktionsmischung, vorzugsweise von etwa 45°C bis etwa 80°C, in Gegenwart eines Radikalstarters umsetzt, in die angefallene Reaktionsmischung 2,0 bis etwa 2,5 mol, vorzugsweise 2,0 bis etwa 2,1 mol Chlor bei Temperaturen von etwa 0°C bis etwa 100°C, vorzugsweise von etwa 40°C bis etwa 80°C einleitet und anschließend die erhaltene Mischung zur Trockne einengt.

Das erfindungsgemäße Verfahren wird durch das nachstehende Reaktionsschema wiedergegeben:

(1) H₂N-CH₂-CH=CH₂ + 1/2 H₂SO₄ → H₂N-CH₂-CH=CH₂ · 1/2 H₂SO₄

(2) H₂N-CH₂-CH = CH₂ · 1/2 H₂SO₄ + HS-(CH₂)₂-OH → H₂N-(CH₂)₃-S-(CH₂)₂-OH · 1/2 H₂SO₄

(3) H₂N-(CH₂)₃-S-(CH₂)₂-OH · 1/2 H₂SO₄ + 2 Cl₂ + H₂O → H₂N-(CH₂)₃-SO₂-(CH₂)₂-Cl · 1/2 H₂SO₄ + 3 HCl

Die Konzentration der verdünnten (wäßrigen) Schwefelsäure, in welcher die Reaktion vorgenommen wird, bewegt sich zweckmäßigerweise zwischen etwa 10 % und etwa 50 %, vorzugsweise etwa 20 % und etwa 40 %. Bei Konzentrationen >50 % kann es zu Ausfällungen während der Reaktion kommen; Konzentrationen <10 % sind wegen schlechterer Raumzeitausbeuten wirtschaftlich nicht sinnvoll.

Was die Mengenverhältnisse anbelangt, so ist es zweckmäßig, 1,0 mol Allylamin in etwa 0,5 bis etwa 0,6 mol, vorzugsweise etwa 0,50 bis etwa 0,55 mol, verdünnter (wäßriger) Schwefelsäure bei einer Temperatur innerhalb der vorstehend genannten Bereiche, bei welcher der verwendete Radikalstarter eine Halbwertszeit von etwa 1 - 5 Stunden hat, vorzulegen und etwa 0,9 bis etwa 1,5 mol, vorzugsweise etwa 0,95 bis etwa 1,05 mol Mercaptoethanol, wobei etwa 0,1 bis etwa 5,0 g, bevorzugt etwa 0,5 bis etwa 1,5 g Radikalstarter pro mol Mercaptoethanol gelöst sind, zuzudosieren. Es ist auch möglich, Mercaptoethanol vorzulegen, wobei etwa 0,1 bis etwa 5,0 g, bevorzugt etwa 0,5 bis etwa 1,5 g Radikalstarter pro mol Mercaptoethanol gelöst sind und 1,0 mol Allylamin in etwa 0,5 bis etwa 0, 6 mol, bevorzugt etwa 0,5 bis etwa 0,55 mol verdünnter (wäßriger) Schwefelsäure bei einer Temperatur, bei welcher der Radikalstarter eine Halbwertszeit von etwa 1 bis 5 Stunden hat, zuzudosieren. Man kann jedoch auch so verfahren, daß man 1,0 mol Allylamin in etwa 0,5 bis etwa 0,6 mol, bevorzugt etwa 0,50 bis etwa 0,55 mol, verdünnter (wäßriger) Schwefelsäure und etwa 0,1 bis etwa 1,0 mol Mercaptoethanol bei einer Temperatur innerhalb der genannten Bereiche, bei welcher der Radikalstarter eine Halbwertszeit von 1 bis 5 Stunden hat, vorzulegen und etwa 0,1 bis etwa 5,0 g, bevorzugt etwa 0,5 bis etwa 1,5 g Radikalstarter pro mol Mercaptoethanol, im restlichen Mercaptoethanol bzw. in Wasser gelöst, zuzudosieren. Es können auch alle Komponenten gemeinsam vorgelegt werden, jedoch ist dies auf Grund der freiwerdenden Reaktionswärme für eine technische Durchführung kritisch.

Als Radikalstarter sind solche, die im Reaktionsmedium löslich sind, wie beispielsweise 2,2'-Azobis[2-(2-imidazolin-2-yl)propan]dihydrochlorid oder 2,2'-Azobis-(2-amidinopropan)-dihydrochlorid bevorzugt. Es können auch Radikalstarter eingesetzt werden, die mit Schwefelsäure im Reaktionsmedium lösliche Sulfate bilden, wie beispielsweise 2,2'-Azobis[2-(2-imidazolin-2-yl)propan].

Das erfindungsgemäße Verfahren wird zweckmäßigerweise bei Atmosphärendruck durchgeführt; eine Verfahrensdurchführung bei erhöhtem oder vermindertem Druck ist jedoch ebenfalls möglich.

Vorteilhaft an dem erfindungsgemäßen Verfahren ist, daß außer der abdestillierten, wiederverwendbaren Salzsäure keine Abfallprodukte entstehen und die Reaktion als Eintopfverfahren durchgeführt wird, wobei das 3'-Aminopropyl-2-chlorethylsulfon-semisulfat ohne weitere Reinigung in überraschend hoher Ausbeute (>95 % der Theorie) und Reinheit (>98 %) anfällt, wodurch sich das Verfahren aus ökonomischer und ökologischer Sicht sehr günstig gestaltet.

Durch die nachstehenden Beispiele wird die Erfindung näher erläutert, ohne darauf beschränkt zu werden.

### Beispiel 1

In einem 2-l-Vierhalskolben mit Rührer, Tropftrichter, Thermometer und Rückflußkühler werden 400 g Eis und 204 g (2,0 mol) Schwefelsäure 96 %ig vorgelegt. Dann läßt man 228 g (4,0 mol) Allylamin zulaufen. Darauf gibt man 312 g (4,0 mol) Mercaptoethanol zu und heizt den Ansatz auf 55°C auf. Zu der Mischung dosiert man bei 55°C innerhalb von 15 min eine Lösung von 4,0 g 2,2'-Azobis[2-(2-imidazolin-2-yl)propan]-dihyrochlorid in 50 ml Wasser zu. Nach beendeter Reaktion gibt man 300 ml Wasser zu und leitet bei 50°C in 4 Stunden 580 g (8,2 mol) Chlor in die Lösung ein. Anschließend dampft man im Vakuum ein und erhält 1038 g feuchtes, farbloses 3'-Aminopropyl-2-chlorethylsulfon-semisulfat mit einem Gehalt von 87,3 % und einer Reinheit >98 % (nach Elementaranalyse), was einer Ausbeute von 96,5 % der Theorie entspricht.
- IR (KBr):: 875, 1050, 1125, 1210, 1290, 1515, 1630, 2920, 2980, 3070, 3200 cm⁻¹.
- ¹H-NMR (D₆DMSO):: δ = 2.03 (tt, J = 7.0 Hz; 2H, CH₂-CH₂-CH₂), 2.93 (m; 2H, CH₂-NH₃⁺), 3.35 (t, J = 7.0 Hz; 2H, SO₂-CH₂-CH₂-Cl), 3.96 (t, J = 7.0 Hz; 2H, CH₂-Cl), 8.0 (m, breit; 3H, NH₃⁺).

| Elementaranalyse (feuchtes Produkt, 87 %ig): | | |
|---|---|---|
| | Ber.: | Gef.: |
| C | 22,3 % | 22,4 % |
| N | 5,2 % | 5,2 % |
| S | 17,9 % | 17,9 % |
| Cl (organisch) | 13,2 % | 13,0 % |

### Beispiel 2

In einem 2-l-Vierhalskolben mit Rührer, Tropftrichter, Thermometer und Rückflußkühler werden 400 g Eis und 204 g (2,0 mol) Schwefelsäure 96 %ig vorgelegt. Dann läßt man 228 g (4,0 mol) Allylamin zulaufen. Darauf gibt man 312 g (4,0 mol) Mercaptoethanol zu und heizt den Ansatz auf 70°C auf. Zu der Mischung dosiert man bei 70°C innerhalb von 15 min eine Lösung von 4,0 g 2,2'-Azobis(2-amidinopropan)dihydrochlorid in 50 ml Wasser zu. Nach beendeter Reaktion gibt man 300 ml Wasser zu und leitet bei 50°C in 4 Stunden 580 g (8,2 mol) Chlor in die Lösung ein. Anschließend dampft man im Vakuum ein und erhält 1009 g feuchtes 3'-Aminopropyl-2-chlorethylsulfon-semisulfat mit einem Gehalt von 89,8 % und einer Reinheit von >98 % (nach Elementaranalyse), was einer Ausbeute von 96,1 % der Theorie entspricht.

Die spektroskopischen Daten sind mit den in Beispiel 1 angegebenen identisch.

## Patentansprüche

1. Verfahren zur Herstellung von 3'-Aminopropyl-2-chlorethylsulfon-semisulfat in sehr guter Ausbeute und hoher Reinheit, dadurch gekennzeichnet, daß man in einem Eintopfverfahren Allylamin mit Mercaptoethanol in etwa 0,5 bis etwa 0,6 mol verdünnter (wäßriger) Schwefelsäure, bezogen auf das Allylamin, bei Temperaturen von etwa 0°C bis zum Siedepunkt der Reaktionsmischung in Gegenwart eines Radikalstarters umsetzt, in die angefallene Reaktionsmischung 2,0 bis etwa 2,5 mol Chlor bei Temperaturen von etwa 0°C bis etwa 100°C einleitet und anschließend die erhaltene Mischung zur Trockne einengt.

2. Verfahren nach Anspruch 1, dadurch gekennzeichnet, daß man die erste Reaktionsstufe bei Temperaturen von etwa 45°C bis etwa 80°C vornimmt.

3. Verfahren nach mindestens einem der Ansprüche 1 und 2, dadurch gekennzeichnet, daß man die Chloreinleitung bei Temperaturen von etwa 40°C bis etwa 80°C vornimmt.

4. Verfahren nach mindestens einem der Ansprüche 1 bis 3, dadurch gekennzeichnet, daß man die Umsetzung des Allylamins mit dem Mercaptoethanol in verdünnter (wäßriger) Schwefelsäure bei der Temperatur, bei welcher der Radikalstarter eine Halbwertszeit von etwa 1 bis etwa 5 Stunden hat, durchführt.

5. Verfahren nach mindestens einem der Ansprüche 1 bis 4, dadurch gekennzeichnet, daß man als Radikalstarter 2,2'-Azobis[2-(2-imidazolin-2-yl)propan]dihydrochlorid verwendet.

6. Verfahren nach mindestens einem der Ansprüche 1 bis 3, dadurch gekennzeichnet, daß man als Radikalstarter 2,2'-Azobis(2-amidinopropan)dihydrochlorid verwendet.

7. Verfahren nach mindestens einem der Ansprüche 1 bis 4, dadurch gekennzeichnet, daß man als Radikalstarter 2,2'-Azobis[2-(2-imidazolin-2-yl)propan] verwendet.

8. Verfahren nach mindestens einem der Ansprüche 1 bis 7, dadurch gekennzeichnet, daß man die Behandlung mit Chlor bei Temperaturen von etwa 40 bis etwa 80°C durchführt.

9. Verfahren nach mindestens einem der Ansprüche 1 bis 8, dadurch gekennzeichnet, daß man die erste Reaktionsstufe in einer etwa 10 bis etwa 50 %igen wäßrigen Schwefelsäure vornimmt.

10. Verfahren nach mindestens einem der Ansprüche 1 bis 9, dadurch gekennzeichnet, daß man die erste Reaktionsstufe in einer etwa 20 bis etwa 40 %igen wäßrigen Schwefelsäure vornimmt.

11. Verfahren nach mindestens einem der Ansprüche 1 bis 8, dadurch gekennzeichnet, daß man 1,0 mol Allylamin mit etwa 0,9 bis etwa 1,5 mol Mercaptoethanol in Gegenwart von etwa 0,1 bis etwa 5,0 g Radikalstarter pro mol Mercaptoethanol zur Umsetzung bringt.

12. Verfahren nach mindestens einem der Ansprüche 1 bis 10, dadurch gekennzeichnet, daß man die Reaktion bei Atmosphärendruck, bei Vakuum oder bei Überdruck durchführt.

## Claims

1. A process for the preparation of 3-aminopropyl 2-chloroethyl sulfone semisulfate in very good yield and high purity, which comprises reacting allylamine with mercaptoethanol in a one-pot process in about 0.5 to about 0.6 mol of dilute (aqueous) sulfuric acid, relative to allylamine, at temperatures of about 0°C up to the boiling point of the reaction mixture in the presence of a free-radical initiator, introducing 2.0 to about 2.5 mol of chlorine into the resultant reaction mixture at temperatures of about 0°C to about 100°C and then concentrating the mixture obtained to dryness.

2. The process as claimed in claim 1, wherein the first reaction step is carried out at temperatures of about 45°C to about 80°C.

3. The process as claimed in at least one of claims 1 and 2, wherein chlorine is passed in at temperatures of about 40°C to about 80°C.

4. The process as claimed in at least one of claims 1 to 3, wherein the reaction of allylamine with mercaptoethanol in dilute (aqueous) sulfuric acid is carried out at the temperature at which the free-radical initiator has a half life of about 1 to about 5 hours.

5. The process as claimed in at least one of claims 1 to 4, wherein the free-radical initiator used is 2,2'-azobis[2-(2-imidazolin-2-yl)propane] dihydrochloride.

6. The process as claimed in at least one of claims 1 to 3, wherein the free-radical initiator used is 2,2'-azobis(2-amidinopropane) dihydrochloride.

7. The process as claimed in at least one of claims 1 to 4, wherein the free-radical initiator used is 2,2'-azobis[2-(2-imidazolin-2-yl)propane].

8. The process as claimed in at least one of claims 1 to 7, wherein the treatment with chlorine is carried out at temperatures from about 40 to about 80°C.

9. The process as claimed in at least one of claims 1 to 8, wherein the first reaction step is carried out in about 10 to about 50% strength aqueous sulfuric acid.

10. The process as claimed in at least one of claims 1 to 9, wherein the first reaction step is carried out in about 20 to about 40% strength aqueous sulfuric acid.

11. The process as claimed in at least one of claims 1 to 8, wherein 1.0 mol of allylamine is brought to reaction with about 0.9 to about 1.5 mol of mercaptoethanol in the presence of about 0.1 to about 5.0 g of free-radical initiator per mol of mercaptoethanol.

12. The process as claimed in at least one of claims 1 to 10, wherein the reaction is carried out at atmospheric pressure, in vacuo or at superatmospheric pressure.

## Revendications

1. Procédé pour la préparation du semisulfate de 3'-aminopropyl-2-chloréthylsulfone que l'on obtient en un très bon rendement et en une pureté élevée, procédé caractérisé en ce que, dans un procédé utilisant un seul réacteur, on fait réagir de l'allylamine avec du mercapto éthanol dans environ 0,5 à environ 0,6 mole d'acide sulfurique dilué (aqueux) par rapport à l'allylamine à des températures d'environ 0°C jusqu'au point d'ébullition du mélange réactionnel, en présence d'un amorceur générateur de radicaux libres, on introduit dans le mélange ainsi obtenu 2,0 à environ 2,5 moles de chlore à des températures d'environ 0°C à environ 100°C, puis on concentre à siccité le mélange obtenu.

2. procédé selon la revendication 1, caractérisé en ce qu'on effectue la première étape de la réaction à des températures d'environ 45°C à environ 80°C.

3. Procédé selon l'une au moins des revendications 1 et 2, caractérisé en ce qu'on effectue l'introduction du chlore à des températures d'environ 40°C à environ 80°C.

4. Procédé selon l'une au moins des revendications 1 à 3, caractérisé en ce qu'on conduit la réaction de l'allylamine avec le mercapto éthanol dans de l'acide sulfurique dilué (aqueux) à la température à laquelle l'amorceur générateur de radicaux libres présente une période de temps de demi vie d'environ 1 à environ 5 heures.

5. Procédé selon l'une au moins des revendications 1 à 4, caractérisé en ce qu'on utilise comme amorceur générateur de radicaux libres le dichlorhydrate de 2,2'-azobis[2-(2-imidazolin-2-yl)propane].

6. Procédé selon l'une au moins des revendications 1 à 3, caractérisé en ce que l'on utilise comme amorceur générateur de radicaux libres le dichlorhydrate de 2,2'-azobis(2-(amidinopropane).

7. Procédé selon l'une au moins des revendications 1 à 4, caractérisé en ce qu'on utilise comme amorceur générateur de radicaux libres le 2,2-azobis[2-(2-imidazoline-2-yl-propane].

8. Procédé selon l'une au moins des revendications 1 à 7, caractérisé en ce qu'on effectue le traitement par le chlore à des températures d'environ 40 à environ 80°C.

9. Procédé selon l'une au moins des revendications 1 à 8, caractérisé en ce qu'on effectue la première étape de la réaction dans un acide sulfurique aqueux contenant environ 10 à environ 50 % (d'acide sulfurique).

10. Procédé selon l'une au moins des reevendications 1 à 9, caractérisé en ce qu'on effectue la première étape de la réaction dans de l'acide sulfurique aqueux à environ 20 jusqu'à environ 40 % (d'acide sulfurique).

11. Procédé selon l'une au moins des revendications 1 à 8, caractérisé en ce qu'on fait réagir 1,0 mole d'allylamine avec environ 0,9 à environ 1,5 mole de mercapto éthanol, en présence d'environ 0,1 à environ 5,0 g d'un amorceur générateur de radicaux libres, par mole de mercapto éthanol.

12. Procédé selon l'une au moins des revendications 1 à 10, caractérisé en ce qu'on effectue la réaction sous la pression atmosphérique, sous une dépression ou sous une surpression.
